# EUROPEAN PATENT SPECIFICATION

(11) **EP 1 389 081 B1**
(45) Date of publication and mention of the grant of the patent: **23.04.2008**
(21) Application number: 02745162.4
(22) Date of filing: 21.05.2002
(51) Int. Cl.: A61F 5/445

(54) **AN OSTOMY APPLIANCE**
OSTOMIE-VORRICHTUNG
ACCESSOIRE POUR STOMIE

(30) Priority: 21.05.2001 DK 200100820
(43) Date of publication of application: 18.02.2004
(73) Proprietor: COLOPLAST A/S, 3050 Humlebaek (DK)
(72) Inventor: HAGSTROEM, Per, DK-2970 H rsholm (DK); BUHL, Anne, Louise, 2950 Vedb k (DK)
(74) Representative: Nilausen, Kim
(86) International application number: PCT/DK2002/000339
(87) International publication number: WO 2002/094143

(56) References cited:
- EP-A- 0 408 296
- EP-A- 0 477 802
- EP-A- 0 985 390
- GB-A- 2 094 153
- GB-A- 2 216 007
- US-A- 5 759 180
- US-A- 5 989 235

## Description

### 1. Field of the Invention

The present invention relates to an ostomy appliance as defined in claim 1.

In connection with surgery for a number of diseases in the gastro-intestinal or urinary tract a consequence is, in many cases, that the colon, the ileum or the ureter has been exposed surgically and the patient is left with an abdominal stoma, or, in nephrostomy or ureterostomy, the ureter or a catheter is exposed in the back or the chest region or abdominal region, and the effluents or waste products of the body, which are conveyed through these organs, are discharged through the artificial orifice or opening and are collected in a collection bag, which is usually adhered to the skin by means of an adhesive wafer or plate having an inlet opening for accommodating the stoma/ureter/catheter. Also in connection with a fistula, the patient will have to rely on an appliance to collect the bodily material emerging from such opening.

Ostomy appliances are well known. Such appliances may be two-piece or one-piece appliances. In both types of appliances, an adhesive barrier member (or base plate) is attached to the wearer's abdomen/back/chest. In case of a one-piece appliance, a receiving member or bag is attached to the base plate. In case of a two-piece appliance, the adhesive barrier member forms part of a body side member and a receiving member or bag is attached releasably to the body side ostomy member for receiving exudates from the stoma.

When using one-piece appliances, the whole appliance, including the adhesive skin barrier securing the appliance to the skin is normally removed and replaced by a fresh appliance. When using two-piece appliances, the body side ostomy member is left in place up to several days, and only the receiving member or bag attached to the body side member is replaced. The attachment means for attaching an ostomy receiving bag may be a system known per se comprising matching coupling rings or matching flanges and adhesive surfaces engaging with and sealing against a flange area of the body side member.

In order to secure discretion, for decorative purposes and providing softness, low noise generation and comfort in wear it has been proposed to provide ostomy receiving bags with a cover or front layer.

### 2. Description of the Related Art

GB Patent Application No. 2 064 333 (Watkins) discloses an ostomy device comprising an outer cover of a non-woven material bonded by a radio frequency welded seam to an inner impermeable pouch and a flange of a non-woven material. The ostomy device disclosed in GB 2 064 333 is claimed to overcome problems that the skin to which conventional flanges is attached to cannot "breathe" and perspire normally. The cover of a non-woven material is bonded to the inner impermeable pouch along the edge. The device disclosed in GB Patent Application No. 2 064 333 suffers from the drawback that it may be difficult to clean due to folding of the cover when wiping using a humid or wet cloth and furthermore, the risk of tearing the cover is quite high. Furthermore, if placed on the side facing the user, the cover will only to a very limited degree assist in venting humidity due to perspiration which may cause nuisance and skin problems. GB 2 064 333 further discloses a composite element compriisng at least one sheet of liquid and odour impermeable plastic film material capable of radio frequency welding bonded at its edges or in discrete separated areas to one or more sheets of a woven or non-woven fibre material in which the fibre and any binding fibre used are non-dielectric. Such element suffers from the same drawbacks as stated above with respect to the risk of tearing the cover when cleaning by wiping using a humid or wet cloth as the edges are not bonded.

U.S. Patent Nos. 5,455,091 and 5,470,624 (Oreglia) disclose a sheet material which comprises a non-woven plastics film and bonded thereto without an adhesive, a barrier film. The film is especially useful for production of ostomy bags and is stated to combine softness, low noise generation, comfort in wear, light weight and mechanical strength and is laminated by heat lamination to produce an intimate bond between the films. Such sheet material suffers from the drawback that it is often very stiff due to the lamination and thus may be directly uncomfortable and furthermore, if placed on the side facing the user, the cover will not assist in venting humidity.

US Patent No. 5,759,180 discloses a cover for an ostomy bag which is opaque to camouflage waste in the ostomy bag and which is moisture resistant to prevent deterioration of the cover when exposed to moisture as well as to prevent the cover from separating form the bag when exposed to moisture. The cover may include decorative patterns. The rear side of the cover preferably comprises an adhesive material which is attachable, removable, and reattachable to an ostomy bag. The cover is affixed over substantially all of the ostomy bag and at least a part of the neck portion of the front side of the ostomy bag. The adhesive on the rear side of the cover may be applied all over the rear side or alternatively, the adhesive may be applied at discrete locations on the rear side. The cover disclosed in US Patent No. 5,759,180 suffers from the drawback that, if placed on the side facing the user, the cover will only to a very limited degree assist in venting humidity.

European patent application No. EP 0 408 296 (Schirmer) discloses a comfortable ostomy bag that is made with air cushion film so that the airbubbles are on the bag outside. Such a surface is very difficult to clean in the area between the bubbles and cleaning by wiping using a humid or wet cloth would cause considerable shear which may cause leaks in the sealing to the skin or in a coupling area.

European patent application No. EP 0 985 390 (Falconer) provides an ostomy bag with comfort layers where the comfort layers are secured to the front and rear wall by welding around a peripheral seam. A filter housing is located towards an upper portion of the front wall. The housing is formed by a filter cover, which is secured through the front comfort layer to the front wall.

The filter cover may be attached by welding, and the heat generated by welding is sufficient to at least partly melt the comfort layer in order to perform a reliable seal between the filter cover and the front wall.

Thus there is still a need for ostomy appliances showing improved performance with respect to easiness to clean without folding and tearing and improved properties with respect to venting of humidity in order to prevent skin problems.

The above drawbacks are alleviated by the present invention as stated below.

### SUMMARY OF THE INVENTION

The invention relates to a disposable receiving bag comprising front and rear walls sealed together along the rim and provided with an inlet opening wherein the receiving bag is provided with a cover of a porous material covering one or both surfaces thereof.

### Brief Description of the Drawings

The invention is disclosed more in detail with reference to the drawings in which
Fig. 1 shows a proposed pattern for welding a cover of a porous material to a wall of an ostomy receiving bag of the invention,
Fig 2 shows an alternative pattern for welding a cover of a porous material to a wall of an ostomy receiving bag of the invention,
Fig 3 shows a further pattern for welding an cover of a porous material to a wall of an ostomy receiving bag of the invention,
Fig. 4 shows a still further pattern for welding a cover of a porous material to a wall of an ostomy receiving bag of the invention,
Fig. 5 shows a sectional view of an embodiment of a wall of a receiving bag according to the invention, and
Fig. 6 shows a sectional view of another embodiment of a wall of a receiving bag according to the invention.

### Detailed Description of the Present Invention

The invention relates to a disposable receiving bag comprising front and rear walls sealed to another along the rim and provided with an inlet opening wherein the receiving bag is provided with a cover of a porous sheet material covering one or both surfaces thereof and wherein the cover is secured to the wall or walls of the receiving bag by welding along the rim and in discrete locations over the surface of the cover.

The securing of a cover of a porous sheet material covering one or both surfaces of the receiving bag to the wall or walls of the receiving bag by welding along the rim and in discrete locations over the surface of the cover provides a product which keeps its flexibility while maintaining the resistance against folding and tearing on wiping and shows improved properties with respect to venting of humidity in order to prevent skin problems. This is presumed to be due to the fact that there is a pattern of welding seams in which the two layers are integrated and neighbouring areas in which the layers have been deformed leaving areas between the welding seams where the layers are separated and thus better allows for venting of the area between the skin and the wall of the collecting bag.

Suitable porous materials for use as cover for the purpose of the present invention are woven or non-woven sheet materials which are moisture resistant and may be united with materials conventionally used in the production of ostomy appliances. Such materials are preferably compatible with the materials used for the preparation of ostomy appliances allowing a simple assembling using welding e.g. using heat, laser or high frequency welding.

Preferred materials for use as covers according to the present invention are non-woven materials of polyethylene, polypropylene or a polyester which are thermo-formable and may easily be welded to the wall of the bag using conventional techniques.

The ostomy receiving bag according to the invention may be adapted for use together with an ostomy body side member (2-piece appliance) wherein the receiving bag is provided with coupling means for releasable securing to matching coupling means placed on the ostomy body side member and wherein the inlet opening is adapted for alignment with a hole of the ostomy body side member for receiving a stoma.

The ostomy receiving bag according to the invention may, as an alternative, be adapted for use directly (1-piece appliance) in which case the bag is provided with an adhesive wafer for securing the receiving bag to the user's skin, said bag and wafer having an inlet opening for receiving a stoma.

The receiving bag itself comprising front and rear walls sealed to another along the rim and provided with an inlet opening may be made in analogy with and from materials conventionally used for the preparation of ostomy appliances.

The welding seams are preferably in the form of a regular pattern giving an option of flow of air and transportation of humidity along the surface of the skin.

Thus, it is preferred to provide dense welding seams in which a cover of a porous material is integrally united and deformed leaving the intermediate areas lifted and acting like cushions keeping the wall of the collecting bag free of the skin.
The deformation may be provided during the welding of the cover layer to the wall of the bag leaving the intermediate areas lifted from the wall of the bag or, in the alternative, a cover layer having a honeycomb-pattern may be used, in which case the welding seams are placed in the lines of the pattern

These welding seams may be produced in a manner analogous to the welding of polymer sheets for production of ostomy bags.

In the alternative, a cover layer having a honeycomb-pattern is used and welded to the wall of the bag at the tops of the areas between the lines of the pattern. Such welding may be carried out by laser welding or by conventional welding, preferably from the side of the wall of the bag.

An ostomy body side member for use together with an ostomy receiving bag according to the invention may be produced from standard materials normally used for preparation of disposable ostomy and wound and incontinence devices. Thus, the adhesive wafer may be made from a medical grade barrier adhesives known in the such as the formulation being disclosed, for example in US Patents Nos. 4,367,732, 5,051,259 or 5,714,225, and the body side member is being provided with coupling means matching the coupling means of the receiving bag.

The coupling means for use in connection with the present invention may be any suitable coupling means known per se for coupling of ostomy base plates to ostomy collecting bags, e.g. a mechanical coupling such as matching coupling rings such as the coupling rings disclosed in WO 93/18725 or WO 94/18919 or matching flanges for adhesive connection of the type disclosed in U.S. Patent No. 5,800,415.

A preferred pattern is in the form of a honeycomb pattern having dense welding seams in which the two layers are integrally united and the deformation provided during the welding leaves intermediate areas acting like cushions keeping the wall of the collecting bag free of the skin and thus improving the venting of humidity.

### Description of the Preferred Embodiments

The invention is now explained more in detail with reference to the drawings showing preferred embodiments of the invention.

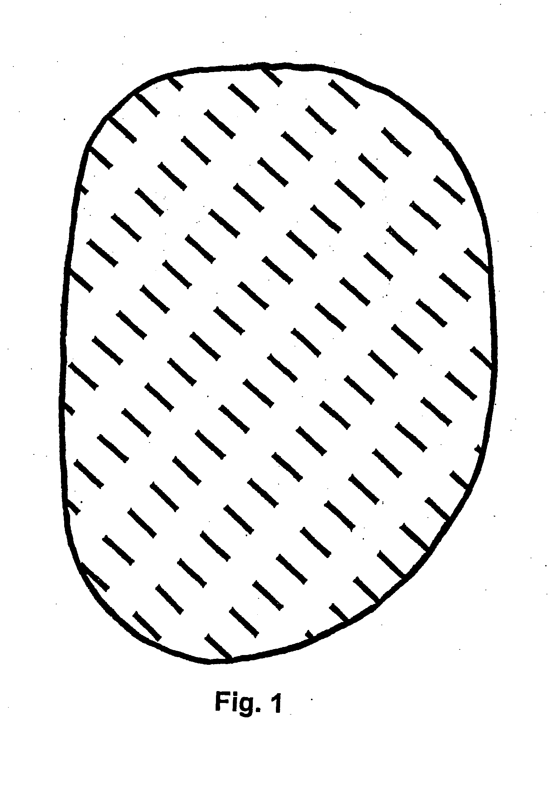

Reference is made to Figure 1 of the drawings showing a proposed pattern for welding a cover of a porous material to the surface of a wall of an ostomy receiving bag of the invention. The pattern is in the form of regularly spaced discrete short welding seams securing the porous layer to the wall of the receiving bag. The pattern allows for venting along the surface of the wall of the bag as this embodiment leaves small ridges of porous material between the welding seams.

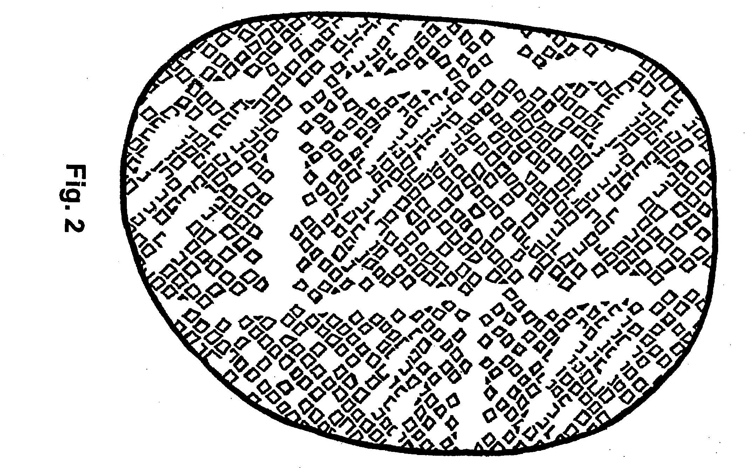

Figure 2 shows an alternative pattern in the form of different welding seams for welding a cover of a porous material to the surface of a wall of an ostomy receiving bag of the invention.

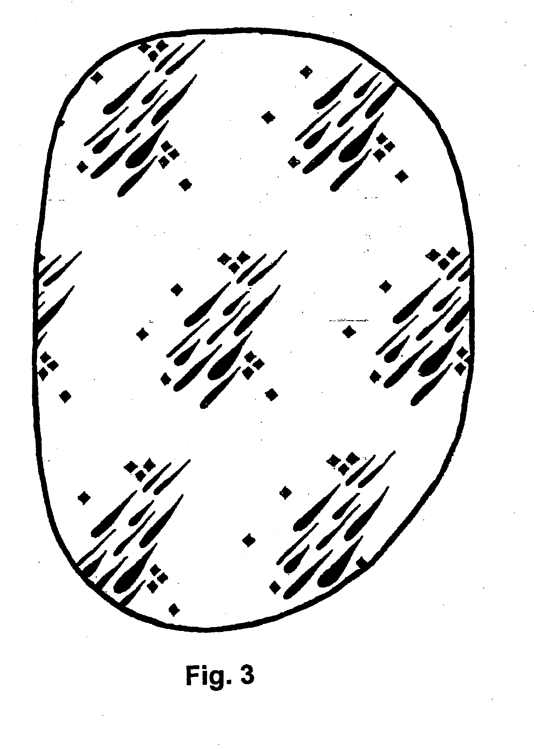

Figure 3 shows a further pattern for welding a cover of a porous material to the surface of a wall of an ostomy receiving bag of the invention. This pattern combines more dense areas with larger areas for more easy venting. In the more dense areas the venting is also secured due to the non-dense welding in these areas.

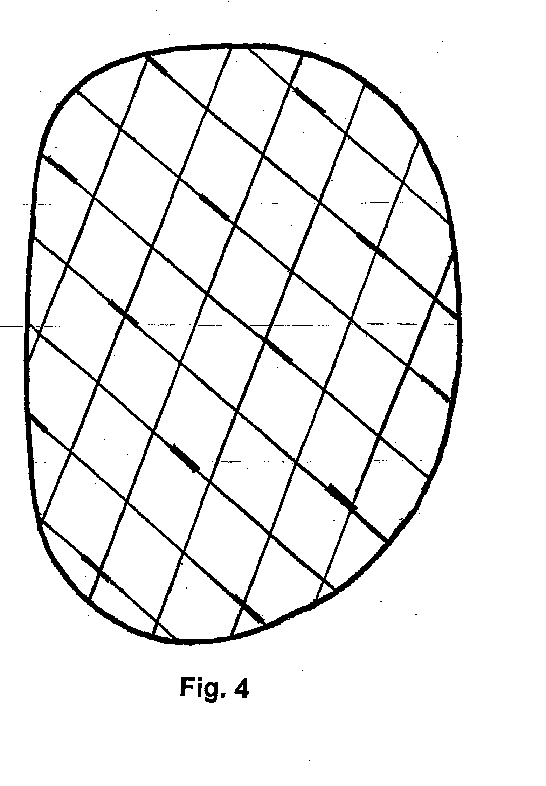

In Figure 4 is shown a preferred pattern for welding a cover of a porous material to the surface of a wall of an ostomy receiving bag of the invention in the form of a rhombus or honeycomb pattern having dense welding seams in which the two layers are integrally united. The intermediate areas between the welding seams are deformed and thus keep the wall of the receiving bag lifted from the skin and thereby improve the venting of humidity from the area covered by the collecting bag without compromising easiness of cleaning by wiping using a humid or wet cloth as no stiff areas protrude from the surface thus being generally smooth. When the cover is provided with a pre-formed honeycomb pattern it may be sufficient to weld the cover to the wall of the bag using spaced discrete short welding seams which is also indicated in Figure 4.

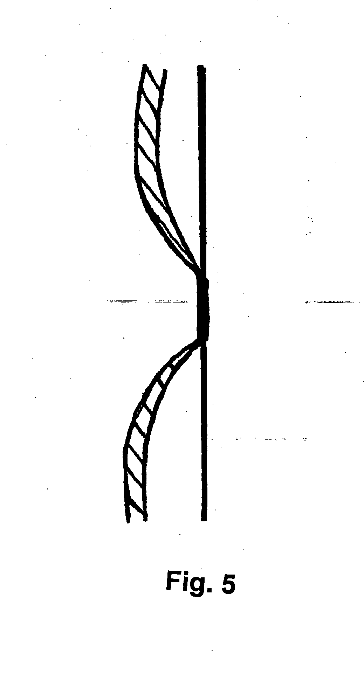

Figure 5 shows a sectional view of an embodiment of a wall of a receiving bag according to the preferred embodiment clearly showing the dense welding seams in which a cover of a porous material is integrally united and deformed leaving the intermediate areas lifted and acting like cushions keeping the wall of the collecting bag free of the skin.

The deformation may be provided during the welding of the cover layer to the wall of the bag leaving the intermediate areas lifted from the wall of the bag or, in the alternative, a cover layer having a honeycomb-pattern may be used, in which case the welding seams are placed in the lines of the pattern.

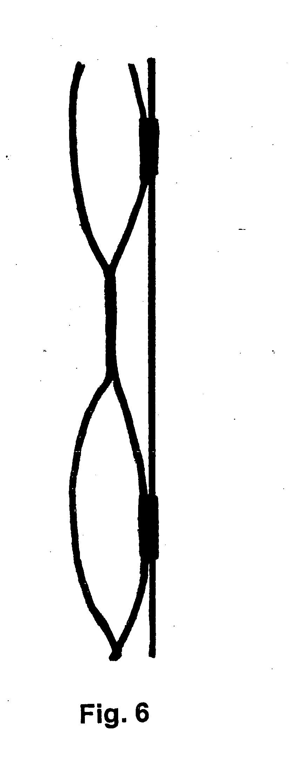

Reference is made to Figure 6 showing another embodiment of a wall of a receiving bag according to the invention in which a cover layer having a honeycomb-pattern is welded to the wall of the bag at the tops of the areas between the lines of the pattern. Such welding may be carried out by laser welding or by conventional welding, preferably from the side of the wall of the bag.

## Claims

1. A disposable receiving bag comprising front and rear walls sealed together along the rim and provided with an inlet opening wherein the receiving bag is provided with a cover of a porous sheet material covering one or both surfaces thereof and wherein the cover is secured to the wall or walls of the receiving bag by welding along the rim **characterised in that** the welding is also in discrete locations over the surface of the cover.

2. An ostomy receiving bag as claimed in claim 1 wherein the receiving bag is provided with coupling means for releasable securing to matching coupling means placed on the ostomy body side member and wherein the inlet opening is adapted for alignment with a hole of an ostomy body side member for receiving a stoma.

3. An ostomy receiving bag as claimed in claim 1 wherein the bag is provided with an adhesive wafer for securing the receiving bag to the user's skin, said wafer having an inlet opening and for receiving a stoma.

4. An ostomy appliance as claimed in any of claims 1 - 3 wherein the welding is in the form of a pattern covering the surface of the cover.

5. An ostomy appliance as claimed in claim 4 wherein the welding is in the form of a regular pattern.

6. An ostomy appliance as claimed in claim 5 wherein the welding is in the form of a honeycomb pattern.

7. An ostomy appliance as claimed any of the claims 1-6, wherein there is a pattern of welding seams in which the cover and the wall are integrated and there are neighbouring areas in which the cover and the wall have been deformed leaving areas between the welding seams where the cover and the wall are separated.

8. An ostomy appliance as claimed any of the claims 1-7, wherein dense welding seams are provided in which the two layers of the cover of porous material are integrally united and deformed leaving the intermediate areas lifted and acting like cushions keeping the wall of the collecting bag free of the skin.

9. An ostomy appliance as claimed any of the claims 1-8, wherein the material for use as a cover is selected from the group consisting of non-woven materials of polyethylene, polypropylene and polyester which are thermo-formable

## Patentansprüche

1. Wegwerfbarer Aufnahmebeutel, der eine vordere Wand und eine hintere Wand aufweist, die längs des Randes dicht miteinander verbunden sind, und mit einer Einlassöffnung versehen ist, wobei der Aufnahmebeutel mit einer Abdeckung aus einem porösen Flachmaterial ausgerüstet ist, die eine seiner Oberflächen oder beide seiner Oberflächen bedeckt, und wobei die Abdeckung durch Verschweißen längs den Rändern an der Wand oder an den Wänden des Aufnahmebeutels befestigt ist, **dadurch gekennzeichnet, dass** die Verschweißung auch an diskreten Stellen auf der Oberfläche der Abdeckung vorliegt.

2. Stoma-Aufnahmebeutel nach Anspruch 1, wobei der Aufnahmebeutel mit einer Verbindungseinrichtung zur lösbaren Befestigung an einer passenden Verbindungseinrichtung versehen ist, die am körperseitigen Stomaelement angeordnet ist, und wobei die Einlassöffnung so ausgebildet ist, dass sie auf ein Loch eines körperseitigen Stomaelements zur Aufnahme eines Stomas ausgerichtet werden kann.

3. Stoma-Aufnahmebeutel nach Anspruch 1, wobei der Beutel mit einem Klebeplättchen zur Befestigung des Aufnahmebeutels an der Haut des Anwenders versehen ist, wobei das Plättchen eine Einlassöffnung zur Aufnahme eines Stomas aufweist.

4. Stomavorrichtung nach einem der Ansprüche 1 bis 3, bei der die Verschweißung in Form eines Musters vorliegt, das die Oberfläche der Abdeckung überdeckt.

5. Stomavorrichtung nach Anspruch 4, bei der die Verschweißung in Form eines regelmäßigen Musters vorliegt.

6. Stomavorrichtung nach Anspruch 5, in der die Verschweißung in Form eines Wabenmusters vorliegt.

7. Stomavorrichtung nach einem der Ansprüche 1 bis 6, bei der ein Muster von Schweißnähten vorliegt, in dem die Abdeckung und die Wand miteinander integriert sind und benachbarte Bereiche vorliegen, in denen die Abdeckung und die Wand verformt wurden und zwischen den Schweißnähten Bereiche freigelassen sind, in denen die Abdeckung und die Wand voneinander getrennt sind.

8. Stomavorrichtung nach einem der Ansprüche 1 bis 7, bei der dicke Schweißnähte vorgesehen sind, in denen die beiden Schichten der Abdeckung auf porösem Material integral miteinander verbunden und so verformt sind, dass sich die dazwischenliegenden Bereiche hochwölben und wie Kissen wirken, welche die Wand des Sammelbeutels von der Haut fernhalten.

9. Stomavorrichtung nach einem der Ansprüche 1 bis 8, bei der das für die Abdeckung verwendete Material aus der Gruppe ausgewählt ist, die aus Vliesmaterialien von Polyethylen, Polypropylen und Polyester, die wärmeformbar sind, besteht.

## Revendications

1. Poche collectrice jetable, comprenant des parois avant et arrière scellées ensemble le long d'une bordure et munie d'un orifice d'entrée, dans laquelle la poche collectrice est munie d'une couverture conçue en une matière feuilletée poreuse, recouvrant une ou les deux surfaces de celle-ci et dans laquelle la couverture est fixée à la paroi ou aux parois de la poche collectrice par une soudure le long de la bordure, **caractérisée en ce que** la soudure est prévue également dans des endroits discrets sur la surface de la couverture.

2. Une poche collectrice pour stomie comme revendiquée dans la revendication 1, dans laquelle la poche collectrice est munie d'un moyen d'accouplement pour permettre la fixation amovible à un moyen d'accouplement s'adaptant sur l'élément du côté corps du patient porteur d'une stomie et dans laquelle l'ouverture d'entrée est adaptée pour assurer l'alignement avec un trou d'un élément du côté corps du patient porteur d'une stomie pour recevoir une stomie.

3. Une poche collectrice pour stomie comme revendiquée dans la revendication 1, dans laquelle la poche est munie d'un pansement adhésif pour fixer la poche collectrice à la peau de l'utilisateur, ledit pansement ayant un orifice d'entrée et étant destiné à recevoir une stomie.

4. Un accessoire pour stomie comme revendiqué dans l'une quelconque des revendications 1 à 3, dans lequel la soudure se présente sous la forme d'un motif couvrant la surface de la couverture.

5. Un accessoire pour stomie comme revendiqué dans la revendication 4, dans lequel la soudure se présente sous la forme d'un motif régulier.

6. Un accessoire pour stomie comme revendiqué dans la revendication 5, dans lequel la soudure se présente sous la forme d'un motif en nid d'abeilles.

7. Un accessoire pour stomie comme revendiqué dans l'une quelconque des revendications 1 à 6, dans lequel il existe un motif de points de soudure dans lequel la couverture et la paroi sont intégrées et il existe des zones adjacentes dans lesquelles la couverture et la paroi ont été malformées, laissant des zones entre les points de soudure où la couverture et la paroi sont séparées.

8. Un accessoire pour stomie comme revendiqué dans l'une quelconque des revendications 1 à 7, dans lequel des gros points de soudure ont été prévus, dans lesquels les deux couches de la couverture faite d'une matière poreuse sont unies intégralement et malformées, laissant les zones intermédiaires surélevées, et agissant comme des amortisseurs, laissant la paroi de la poche collectrice libre de la peau.

9. Un accessoire pour stomie comme revendiqué dans l'une quelconque des revendications 1 à 8, dans lequel la matière destinée à servir de couverture est choisie dans le groupe constitué par les matières non tissées de polyéthylène, polypropylène et polyester, qui sont façonnées à chaud.
